# EUROPEAN PATENT APPLICATION

(11) **EP 4 530 341 A1**
(43) Date of publication of application: **02.04.2025**
(21) Application number: 23382990.2
(22) Date of filing: 28.09.2023
(51) Int. Cl.: C12N 1/14, A23J 3/20, A23L 31/00, A23L 33/10

(54) **FILAMENTOUS FUNGUS BIOMATERIAL, METHOD FOR CULTURING SAID FILAMENTOUS FUNGUS, MYCELIUM-BASED MEAT ANALOGUE AND RESPECTIVE MEAT ANALOGUE ELABORATION METHOD**

(71) Applicant: Libre Foods Biotech, S.L., 08020 Barcelona (ES)
(72) Inventor: Abad, Sergi, 08140 Caldes de Montbui (ES); Reshetnyak, Anne, 08005 Barcelona (ES); Carvalho, Vanessa, 08020 Barcelona (ES)
(74) Representative: Curell Suñol S.L.P.

(57) **Abstract**

The invention refers to a static submerged fermentation process which enhances fungus growth and allows the grown fungus to be collected from the culture media in an easy manner and free of culture media debris. The invention also refers to a filamentous fungus biomaterial obtained with the method, and to a meat analogue elaborated with the filamentous fungus biomaterial and the meat analogue elaboration method.

## Description

### Field of the invention

The invention relates to a filamentous fungus biomaterial, a method for culturing a filamentous fungus, a mycelium-based meat analogue and a meat analogue elaboration method.

### State of the art

Mycelium, owing to its high protein content, low environmental footprint, and versatility, has emerged as a promising ingredient for the food industry. Its main production methods are solid-state fermentation (SSF), and stirred submerged fermentation (StSF).

One significant drawback of SSF for mycelium production is the close association between mycelium and solid substrates (culture media). This entanglement complicates downstream processing and extraction of pure mycelium. The market demands a mycelium material free of substrate debris, as this results in unpleasant experience for the consumers. In SSF, separating mycelium from solid substrates can be labor-intensive and may result in lower mycelium yield. The entanglement often requires mechanical or enzymatic methods for effective separation, potentially impacting the quality and purity of the final product.

Achieving uniform growth and consistent mycelium quality in SSF can be challenging due to variations in substrate composition and mycelial distribution. This can lead to batch-to-batch variations in mycelium-based food products. Furthermore, scaling up SSF processes for commercial mycelium production may be hindered by the need for efficient downstream processing steps.

Developing scalable technologies that minimize the entanglement issue is crucial for large-scale production. StSF offers several advantages for mycelium production. It involves the cultivation of fungi in a liquid medium with continuous agitation, which prevents the high entanglement of mycelial fibers with substrates found in SSF. Contrariwise, this approach also hinders the self-entanglement of mycelium fibers, thereby reducing the texture and strength of mycelium when grown under StSF conditions.

Mycelium in StSF is easily separated from the liquid medium using filtration or centrifugation, resulting in higher mycelium yields. On the other hand, StSF provides better control over growing conditions, leading to more consistent mycelium quality and reduced batch-to-batch variations in mycelium-based food products. StSF processes are easily scalable for commercial production, making it a cost-effective choice for mycelium production.

SSF presents challenges due to mycelium entanglement with solid substrates, StSF offers a cost-effective and efficient alternative for mycelium production in the food industry, with reduced texture features. The choice between these methods should consider the desired characteristics of the final product and the scale of production. Innovations in SSF technology and continued research will further enhance the feasibility and sustainability of mycelium-based foods.

### Summary of the invention

An object of the invention is a filamentous fungus biomaterial, characterized in that it comprises:
- less than 5% of culture media debris
- fungus filaments, an average length of said filaments being between 1 and 1,5 cm
- a water content comprised between 60 and 95%
- a thickness between 0.5 and 10 mm just after harvesting it.

The invention further includes a number of preferred features that are object of the dependent claims and the utility of which will be highlighted hereinafter in the detailed description of an embodiment of the invention.

Preferably, it comprises less than 1% of culture media debris.

Preferably, it comprises a water content of between 70 and 90%.

Preferably, when it presents about 80% of water content, it presents a density of between 1.025 and 1.25 g/cm³.

Preferably, said filamentous fungus biomaterial comprises a strain selected from the group consisting of *Aspergillus oryzae, Aspergillus glaucus, Cordyceps sinensis, Fusarium venenatum (Quom), Ganoderma lucidum (Reishi), Laetiporus sulphureus, Monascus purpureus, Neurospora intermedia, Neurospora crassa, Penicillium nalgiovense, Penicillium chrysogenum, Rhizopus oryzae, Rhizopus chinensis, Rhizopus nigricans, Rhizopus oligosporus, Ustilago maydis, Agaricus campestris, Agaricus sylvaticus, Agrocybe aegerita (cylindracea), Amanita caesarea (open volva), Amanita ponderosa, Boletus aereus, Boletus edulis, Boletus pinophilus (pinicola), Boletus reticulatus (aestivalis), Calocybe gambosa, Cantharellus cibarius, Cantharellus cinereus, Cantharellus lutescens, Cantharellus tubaeformis, Clitocybe geotropa, Craterellus cornucioioides, Fistulina hepatica, Hygrocybe pratensis, Hydnum albidum, Hydnum repandum, Hydnum rufescens, Hygrophorus agathosmus, Hygrophorus gliocyclus, Hygrophorus latitabundus (limacinus), Hygrophorus marzuolus, Hygrophorus penarius, Hygrophorus russula, Lactarius delicious, Lactarius quietcolor, Lactarius salmonicolor, Lactarius sanguifluus, Lactarius semisanguifluss, Lepista panaeolus (luscina), Lepista nuda, Lepista personata, Macrolepiota procera, Marasmius oreades, Pleurotus eryngii, Pleurotus ostreatus, Rhizopogon luteolus (obtextus), Rhizopogon roseolus, Russula cyanozantha, Russula virescens, Suillus luteus, Terfezia arenaria, Terfezia claveryi, Terfezia leptoderma, Tricholoma portentosum, Tricholoma terreum, Tuber aestivum, Tuber borchii, Tuver brumale, Tuber indicum, Tuber magnatum, Tuber melanosporum (nigrum), Ustilago maydis, Xerocomus badius (Boletus badius), Agaricus arvensis, Agaricus bisporus, Agaricus bitorquis, agaricus blazei, Agaricus brunnescens, Agrocybe aegerita (cylindracea) Auricularia auriculaa-judae, Auricularia polytricha, Coprinus comatus, Flammulina velutipes, Grifola frondose, Hericium erinaceus, Lentinula erodes, Hypsizygus marmoreus, Hypsizygus tessulatus, Pholiota nameko, Pleurotus cystidiosus, Pleurotus cornucopiae (citrinopileatus), Pleurotus djamor, Pleurotus fabellatus, Pleurotus nebrodensis, Pleurotus pulmonarius, Pleurotus sajor-caju, Pleurotus tuber-regium, Sparassis crispa, Stropharia rugosoannulata, Tremella fuciformis, Tremella mesenterica, Tricholoma caligatum (matsutake), Volvariella volvacea, Helvella sp,* and *More hell a sp.*

More preferably, said strain is selected from the group consisting of *Aspergillus oryzae, Aspergillus glaucus, Cordyceps sinensis, Ganoderma lucidum (Reishi), Monascus purpureus, Neurospora intermedia, Neurospora crassa, Penicillium nalgiovense, Penicillium chrysogenum, Rhizopus oryzae, Rhizopus chinensis, Rhizopus nigricans, Ustilago maydis, Agaricus bisporus (Portobello), Aspergilus orizae, Fusarium venenatum (Quom), Laetiporus sulphureus, Pleurotus ostreatus (oyster mushroom),* and *Rhizopus oligosporus.*

Another object of the invention is a method for culturing a filamentous fungus comprising the following steps: a) providing a culture media, said culture media comprising a carbon source and a nitrogen source, wherein the ratio in dry weight between said carbon source and said nitrogen source is comprised between 0.5 and 50; b) inoculating said culture media with a mycelium-based strain to obtain an inoculated media; c) conducting static submerged fermentation of said inoculated media in a sealed tray, wherein before fermentation starts a ratio between air volume and inoculated media volume in said tray is comprised between 0.025 and 0.12, d) harvesting a filamentous fungus biomaterial.

The static submerged fermentation method (also known as submerged unstirred fermentation, SUF) minimizes water consumption compared to traditional submerged fermentation methods, as the mycelium grows in a relatively low-volume liquid medium, where the mycelium itself becomes the dominant component. Thus, it contributes to more sustainable mycelium production by conserving water resources. This reduced volume of liquid translates to lower energy requirements for heating, cooling, and maintaining the fermentation environment, contributing to energy efficiency.

Furthermore, with this method, the mycelium is exposed to a higher concentration of nutrients, promoting efficient nutrient utilization and potentially enhancing mycelium yield and quality. In fact, the mycelium produced with this method often exhibits a desirable texture that can mimic the mouthfeel of traditional meat or other protein sources. This texturized mycelium enhances the versatility of mycelium-based food products.

The mycelium obtained with this method is largely free from solid substrate debris. This simplifies the separation process and results in purer mycelium, reducing the need for extensive cleaning and processing.

Preferably, said method further comprises a step e) of rinsing said filamentous fungus biomaterial with water to obtain a cleaner filamentous fungus biomaterial.

More preferably, said method further comprises a step f) of inactivating said filamentous fungus biomaterial, said step of inactivating said filamentous fungus biomaterial being performed at a temperature comprised between 60 °C and 100 °C for a duration of time comprised between 1 minute to 120 minutes, preferably said temperature is between 60 and 80 °C and said duration of time is between 25 and 35 minutes. Therefore, said filamentous fungus is inactivated.

Preferably, said step c) of conducting static submerged fermentation is carried out at a temperature between 0 °C and 55 °C, more preferably between 20 °C and 40 °C, even more preferably between 25 °C and 35 °C. This is the optimal range of temperature for the fungus to grow.

Preferably, said step c) of conducting static submerged fermentation is carried out for a duration of time comprised between 10 and 120 hours, more preferably between 40 and 90 hours, even more preferably between 70 and 80 hours. This is the optimal range of time for the fungus to grow.

Preferably, said culture media comprises an initial pH between 2 and 9, preferably between 4 and 7, and even more preferably between 5 and 6.

Preferably, said mycelium-based strain used to inoculate said culture media is in the type of spores, a concentration of said mycelium-based strain being between 2×10⁴ and 2×10⁷ spores per ml of culture media.

Preferably, said mycelium-based strain used to inoculate said culture media is in the type of pellets, said pellets representing at least 5% of the total liquid volume of the culture media, more preferably 10% of the total liquid volume of the culture media.

Preferably, said ratio in dry weight between said carbon source and said nitrogen source is comprised between 0.5 to 1.

Preferably, before fermentation starts, said ratio between air volume and culture media volume in said tray is comprised between 0.0375 and 0.08, more preferably between 0.01 and 0.03. This ratio allows to perform static submerged fermentation, as there is no need to provide aeration or additional moisture during the fermentation process.

In a preferred embodiment, a contact surface between said culture media and said air is between 1 and 3 cm²/ml of culture media. This allows the fungus to grow in a way that it allows the filamentous fungus biomaterial to be separated from the culture media in a clean and easy manner.

In another preferred embodiment, a thickness of said culture media is between 1 and 15 mm, preferably between 2.5 and 7.5 mm. This is the thickness that has been found to be optimal to enhance fungus growth in a way that it allows the filamentous fungus biomaterial to be separated from the culture media in a clean and easy manner. Preferably, said carbon source is selected from the group comprising sucrose, oat flour, yeast extract and a combination thereof.

Preferably, said culture media comprises ingredients that enhance fungi growth, said ingredients being selected from the group comprising (NH4)2SO4, KH2P04, MgS04·7H2O, NaCl, CaCl2·2H20.

Preferably, said culture media further comprises a tracer metal solution selected from the group comprising iron sulfate, copper sulfate and zinc sulfate. This enhances fungus respiration.

Preferably, said culture media comprises insoluble particles, wherein at least 80% of said insoluble particles have a particle size of between 20 and 500 µm, preferably between 60 and 150 µm. This particle size enables the fungus to grow properly and in a manner that allows the filamentous fungus biomaterial to be separated from the culture media easily, this is, free of culture media debris.

Preferably, said insoluble particles are complex nitrogen source particles, such as cereals (for example, oats), Fabaceae (for example, soya beans and lentils), or yeast extract.

In another embodiment, said insoluble particles are inorganic nitrogen source particles.

Preferably, said mycelium-based strain is selected from an edible strain of the group consisting of Ascomycotal, Basidiomycota and Mucoromycota division.

Preferably, said mycelium-based strain is selected from an edible strain of the group consisting of Aspergillus and Rhizopus.

Preferably, said mycelium-based strain is selected from the group consisting of *Aspergillus oryzae, Aspergillus glaucus, Cordyceps sinensis, Fusarium venenatum (Quom), Ganoderma lucidum (Reishi), Laetiporus sulphureus, Monascus purpureus, Neurospora intermedia, Neurospora crassa, Penicillium nalgiovense, Penicillium chrysogenum, Rhizopus oryzae, Rhizopus chinensis, Rhizopus nigricans, Rhizopus oligosporus, Ustilago maydis, Agaricus campestris, Agaricus sylvaticus, Agrocybe aegerita (cylindracea), Amanita caesarea (open volva), Amanita ponderosa, Boletus aereus, Boletus edulis, Boletus pinophilus (pinicola), Boletus reticulatus (aestivalis), Calocybe gambosa, Cantharellus cibarius, Cantharellus cinereus, Cantharellus lutescens, Cantharellus tubaeformis, Clitocybe geotropa, Craterellus cornucioioides, Fistulina hepatica, Hygrocybe pratensis, Hydnum albidum, Hydnum repandum, Hydnum rufescens, Hygrophorus agathosmus, Hygrophorus gliocyclus, Hygrophorus latitabundus (limacinus), Hygrophorus marzuolus, Hygrophorus penarius, Hygrophorus russula, Lactarius delicious, Lactarius quietcolor, Lactarius salmonicolor, Lactarius sanguifluus, Lactarius semisanguifluss, Lepista panaeolus (luscina), Lepista nuda, Lepista personata, Macrolepiota procera, Marasmius oreades, Pleurotus eryngii, Pleurotus ostreatus, Rhizopogon luteolus (obtextus), Rhizopogon roseolus, Russula cyanozantha, Russula virescens, Suillus luteus, Terfezia arenaria, Terfezia claveryi, Terfezia leptoderma, Tricholoma portentosum, Tricholoma terreum, Tuber aestivum, Tuber borchii, Tuver brumale, Tuber indicum, Tuber magnatum, Tuber melanosporum (nigrum), Ustilago maydis, Xerocomus badius (Boletus badius), Agaricus arvensis, Agaricus bisporus, Agaricus bitorquis, agaricus blazei, Agaricus brunnescens, Agrocybe aegerita (cylindracea) Auricularia auriculaa-judae, Auricularia polytricha, Coprinus comatus, Flammulina velutipes, Grifola frondose, Hericium erinaceus, Lentinula erodes, Hypsizygus marmoreus, Hypsizygus tessulatus, Pholiota nameko, Pleurotus cystidiosus, Pleurotus cornucopiae (citrinopileatus), Pleurotus djamor, Pleurotus fabellatus, Pleurotus nebrodensis, Pleurotus pulmonarius, Pleurotus sajor-caju, Pleurotus tuber-regium, Sparassis crispa, Stropharia rugosoannulata, Tremella fuciformis, Tremella mesenterica, Tricholoma caligatum (matsutake), Volvariella volvacea, Helvella sp,* and *More hell a sp.*

More preferably, said strain is selected from the group consisting of *Aspergillus oryzae, Aspergillus glaucus, Cordyceps sinensis, Ganoderma lucidum (Reishi), Monascus purpureus, Neurospora intermedia, Neurospora crassa, Penicillium nalgiovense, Penicillium chrysogenum, Rhizopus oryzae, Rhizopus chinensis, Rhizopus nigricans, Ustilago maydis, Agaricus bisporus (Portobello), Aspergilus orizae, Fusarium venenatum (Quom), Laetiporus sulphureus, Pleurotus ostreatus (oyster mushroom),* and *Rhizopus oligosporus.*

Preferably, at least 95% of said filamentous fungus biomaterial is free of culture media debris, more preferably at least 99% of said filamentous fungus biomaterial is free of culture media debris. Therefore, it is possible to obtain a very pure filamentous fungus biomaterial.

Preferably, said filamentous fungus biomaterial presents a water content of between 60 and 95 %. More preferably, said water content is between 89 and 94 % after rinsing and draining said filamentous fungus material with a mesh.

In another embodiment, said water content of said filamentous fungus biomaterial is between 65 to 70% after having applied pressure to said filamentous fungus biomaterial.

Preferably, just after being collected, said filamentous fungus biomaterial presents a thickness of between 0.3 mm and 12 mm, preferably between 0.5 and 10 mm.

Preferably, just after collecting said filamentous fungus biomaterial, filaments of said filamentous fungus biomaterial present an average filament length comprised between 1 cm and 1.5 cm.

Another object of the invention is a mycelium-based meat analogue, characterized in that said meat analogue comprises, with respect of the total weight of said meat analogue:
- between 20 and 90% of a filamentous fungus biomaterial according to the invention;
- between 5 and 15 % of plant-based protein
- between 5 and 15 % of at least one texturing agent.

Preferably, it further comprises a content of between 0.25 and 1.5% of aromatic agents.

Preferably, said at least one texturing agent is selected from the group comprising starch, alginates and carrageenans.

Preferably, said percentage of plant-based protein is between 7.5 and 12.5 % of the total weight of said meat analogue.

Preferably, said percentage of filamentous fungus biomaterial is between 70 and 90 % of the total weight of said meat analogue, obtaining therefore a mycelium-based meat analogue more reminiscent of meat.

Preferably, said percentage of plant-based protein is between 7.5 and 12.5 % of the total weight of said meat analogue.

Preferably, said mycelium-based meat analogue is a chicken meat analogue.

Preferably, said filaments of said filamentous fungus biomaterial present an average filament length comprised between 1 cm and 1.5 cm.

Preferably, said mycelium-based meat analogue contains less than 5% of culture media debris, more preferably less than 1 % of culture media debris.

Another object of the invention is a meat analogue elaboration method, characterized in that it comprises the following steps:
a) combining a filamentous fungus biomaterial according to the invention with at least one plant-based protein source and with at least one ingredient selected from the group consisting of non-starch polysaccharide, starch, vegetable oil and vegetable fat, to create a mycelium-based matrix
b) allowing said mycelium-based matrix to set, forming a mycelium-based meat analogue.

Preferably, it comprises a further step of adding at least one flavour enhancing ingredient.

More preferably, said flavour enhancing ingredient is selected from the group comprising salt, pepper, garlic powder and other spices and seasoning.

Preferably, after step b) of allowing said mycelium-based matrix to set, a further step of cooking said mycelium-based matrix is performed at a temperature of between 60 and 95 °C.

Preferably, said plant-based protein source is selected from the group comprising pea, rice, potato, and bean protein and a combination thereof, more preferably said plant-based protein is pea protein. Pea protein has a high contain of protein, allows creating an emulsion, and has little flavour and smell.

Likewise, the invention also includes other features of detail illustrated in the detailed description of an embodiment of the invention and in the accompanying figures.

### Brief description of the drawings

Further advantages and features of the invention will become apparent from the following description, in which, without any limiting character, preferred embodiments of the invention are disclosed, with reference to the accompanying drawings in which:
Figure 1 is a picture of an *Aspergillus oryzae* fungal matt across a plate according to example 1.
Figure 2 is a picture of a smooth sheet of *Aspergillus oryzae* mycelium according to example 1.
Figure 3 is a picture of *Pleurotus ostratus* hyphae (also referred to as filamentous) monolayer according to example 2.
Figure 4 is a picture of *Ganoderma lucidum* hyphae monolayer according to example 3.
Figure 5 is a picture of *Pleourotus ostreatus* growth after incubation according to example 4.
Figure 6 is a picture of *Ganoderma lucidum* growth after incubation according to example 5.
Figure 7 is a picture of *Ganoderma lucidum* growth after incubation according to example 6.

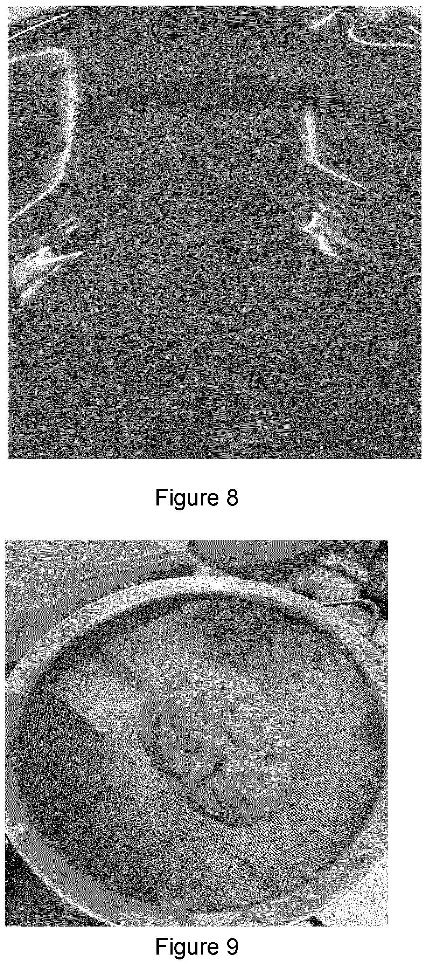

Figure 8 is a picture of a biomaterial obtained after a stirred submerged fermentation (StSF), which is a state of the art process.
Figure 9 is a picture of the biomaterial of Figure 8 after having been drained.

### Detailed description of embodiments of the invention

In the present patent application, the term "static submerged fermentation" (also referred to as submerged unstirred fermentation, SUF) refers to a fermentation process that is carried out without moving or stirring the inoculated media and without aeration or supplying additional moisture. This is particularly important to let the fungus grow in a manner that will form a layer of filamentous biomaterial on top of the culture media. This biomaterial will be able to be separated easily from the culture media. Therefore, obtaining a filamentous fungus biomaterial which contains very little culture media debris.

In the present patent application, the term "pellet" refers to a specific growth morphology or structure formed by the fungal biomass during the fermentation process. Filamentous fungi are known for their multicellular, filament-like structures called hyphae. These hyphae can aggregate and clump together to form dense, spherical or irregularly shaped masses, which are referred to as pellets. Also, the term "flavour enhancing ingredient" must be understood in a broad sense, i.e. it refers to any ingredient that enhances either smell or flavour, or both.

In one embodiment of the invention, the culture of a filamentous fungus is as follows. First, an inoculation material must be prepared in advance to obtain enough spores or mycelia to start a new culture. The inoculation material can be in the type of spores, pellets or any other geometry. In the case of spores, between 2×10⁴ and 2×10⁷ spores must be used to inoculate 1 ml of culture media. In the case of pellets, 10% of the total liquid volume of the culture media must be pellets.

Then, the inoculation material is introduced to a culture media, which has been previously prepared, sterilized and stored under aseptic conditions at 4 °C, obtaining therefore an inoculated media. The culture media is a nutrient-rich culture media and can comprise carbon sources, nitrogen sources, vitamins, and essential minerals. It is important to mention that the culture media comprises insoluble particles that function as a micro-scaffold material, thereby facilitating fungal growth and hypha entanglement. The size of these insoluble particles is comprised between 20 and 500 µm. The size of these particles plays an important role in how the fungus grows. In fact, this range has been found to be optimal as it allows the fungus to grow in a manner that is later easily harvested.

The inoculated media is then evenly distributed inside a sealed tray. Other containers can be used as long as they can be sealed so air or moist doesn't enter the container, as this is key to perform a static submerged fermentation. Furthermore, the containers can be made of several materials, such as plastic or metal.

The sealed tray containing the inoculated media is then placed in an incubation room with controlled temperature and humidity, and it is left in the incubation room unstirred and with no movement at all until cultivation (also referred to as fermentation) is over. It is important to mention that during cultivation there is no additional air or moist supplementation. This unique cultivation method is referred hereinafter as static submerged fermentation, and it allows the mycelium to develop a unique texture and flavour profile. The static submerged fermentation method is carried out at a temperature between 25 and 35 °C, and for a duration of time between 70 and 80 hours. Furthermore, as the tray won't be opened until fermentation is completed, a ratio between air volume and culture media volume is an important parameter and has to be between 0.01 and 0.3. Another important parameter is the contact surface between the culture media and the air contained in the tray, as if the contact surface is not enough, the fungus won't grow as desired. This contact surface has to be between 1 and 3 cm²/ml of culture media.

During the cultivation time, the mycelium proliferates and intertwines, forming a cohesive and filamentous structure reminiscent of meat tissue, specifically reminiscent of chicken breast tissue.

Following the completion of fermentation, the tray is opened and a layer of grown mycelium (also referred to as filamentous fungus biomaterial) formed on the culture media is collected and rinsed with water. Thanks to the size of the insoluble particles and the static submerged fermentation method. Thus, the filamentous fungus biomaterial can be easily separated from the culture media, obtaining therefore a filamentous fungus biomaterial with a very low content of culture media debris.

Finally, the filamentous fungus biomaterial is deactivated at a temperature between 60 and 80 °C and for a duration of time between 25 and 35 minutes.

Once the filamentous fungus biomaterial has been deactivated, it can be combined with other ingredients to form a meat analogue.

### Example 1 (using Aspergillus oryzae):

### Inoculum preparation:

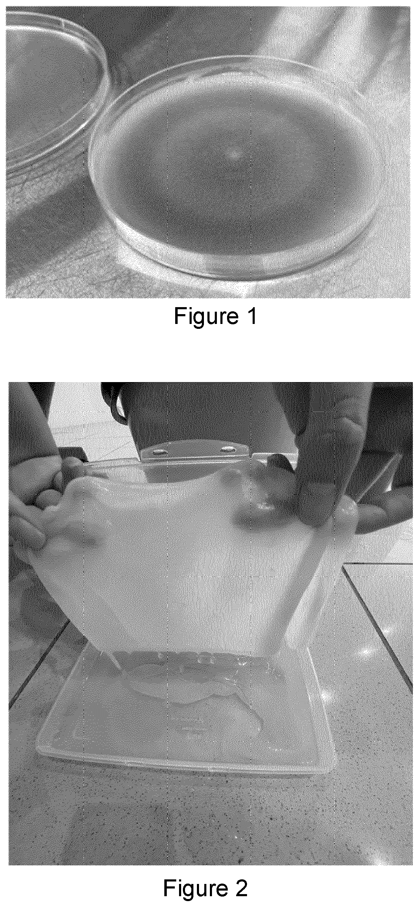

- Fungal spores were inoculated on PDA media by spot inoculation and incubated during 3 to 5 days at 30 °C, until they formed a fungal mat across the plate (see Figure 1).
- Under a laminar air flow chamber, aseptic conditions were maintained and distilled water was slowly poured over the sporulated plates and gently scraped using a Drigalski spatula to collect all the spores in a solution.
- This was decanted into a bottle in order to store the "spore solution".
- The spores were quantified under microscope and calculations were made to determine how much inoculum to add in the growth media, with a concentration of approximately 4×10⁴ spores/ml in the final solution.

### Material preparation:

- Culture media was prepared by taking 20 g/l of oat and 10 g/l of sucrose in 800 ml of distilled water. Over 24 I of this media was autoclaved and cooled prior to inoculation of one batch.
- The trays used were wide based rectangular plastic trays with corresponding plastic lids. The trays were autoclaved after covering them in wrapping paper and kept sterile prior to use.

### Tray inoculation and growth conditions:

- In a sterile environment, the culture media was homogenized prior to inoculation.
- The inoculum was added to the culture media according to the calculations already made.
- This culture media was further homogenized prior to inoculation into the trays.
- 300 ml of culture media were carefully measured before adding into each individual tray and closed tight using the plastic lids.
- The trays were stacked over each other in columns of 10 trays and placed in an incubation chamber.
- The trays were incubated at an average temperature of 30 °C during an incubation period of 3 days.

### Mycelium recovery:

- After incubation, the trays were opened and there was an observable growth of mycelium as a monolayer sheet structure. Each mycelium was a smooth sheet that could be gently removed with gloves to separate it from the underlying liquid substrate (see Figure 2).
- The mycelial sheets collected were gently rested on a colander to remove excess culture media.
- The mycelial sheets were gently washed using filtered water to remove any excess substrate sticking on their surfaces.
- The mycelial sheets were then subjected to a heat shock treatment for RNA reduction. This was achieved by collecting the mycelia in a bag containing a water such that there was a homogenous mixture of mycelia and water. This was placed in a hot water bath and heated at a temperature of 73 °C for 35 minutes.
- The mycelia were strained through a colander and cold water was run through it to complete the heat shock treatment.
- The global weight was measured using a weighing balance and 1 g of a sample was used to measure humidity in a humidity meter.

### Results:

The average mycelial weight collected from this process was quantified to around 250 grams after deactivation, with global weight ranging anywhere between 200 - 320 grams.
The average humidity in the samples was around 80%, within a range of 78-82%. This did not account for any variation in handling during the recovery process.
On average, this process enabled the production of 13 g of wet mycelium / l of mycelium.

### Example 2 (using Pleurotus ostreatus):

In this example, a detailed procedure for growing *Pleurotus ostreatus* (oyster mushroom) using static submerged fermentation in Erlenmeyer flasks with a liquid medium is described.

### Materials and equipment:

- *Pleurotus ostreatus* spores or mycelium culture
- Erlenmeyer flasks (250 ml or 500 ml)
- Glucose
- Citric acid monohydrate
- ZnSO4·7H2O
- Fe(NH4)2(SO4)2·6H2O
- CuSO4·5H2O
- MnSO4·4H2O
- H3BO3
- Na2MoO4·2H2O
- Distilled water
- Autoclave or pressure cooker
- pH meter
- Incubator

### Procedure:

1. Preparation of liquid medium:
   - Weigh the following components and add them to a beaker: 5 g of glucose, 5 g of citric acid monohydrate, 5 g of ZnSO4·7H2O, 1 g of Fe(NH4)2(SO4)2·6H2O, 0.25 g of CuSO4·5H2O, 0.05 g of MnSO4·4H2O, 0.05 g of H3BO3, 0.05 g of Na2MoO4·2H2O.
   - Add distilled water to make up to approximately 95 ml (up to 100 ml) to ensure that all components are dissolved.
   - Adjust the pH of the medium to around 6.0 using a pH meter. Adjust with either HCl or NaOH as necessary.
   - Pour the prepared liquid medium into Erlenmeyer flasks, leaving some headspace to prevent overflow during sterilization.
2. Sterilization:
   - Cover the Erlenmeyer flasks with aluminum foil or cotton plugs.
   - Autoclave the flasks at 121 °C for 15-20 minutes to sterilize the medium. Ensure that the medium is properly sterilized to prevent contamination.
3. Inoculation:
   - Allow the sterilized medium to cool down to room temperature in a sterile environment, such as a laminar flow hood.
   - Aseptically inoculate each flask with a small amount of *Pleurotus ostreatus* spored or mycelium. Use a sterile loop or needle to transfer the inoculum to the medium.
4. Incubation:
   - Seal the flasks with sterile cotton plugs or aluminum foil Incubate the flasks in an incubator at the optimal temperature for *Pleurotus ostreatus* growth, which typically is around 25-28 °C.
5. Monitoring and growth assessment:
   - Periodically monitor the growth of *Pleurotus ostreatus* in the liquid medium. Growth can be assessed visually by observing changes in the medium's appearance, such as mycelial growth, which may appear as white, cottony masses.

### Results:

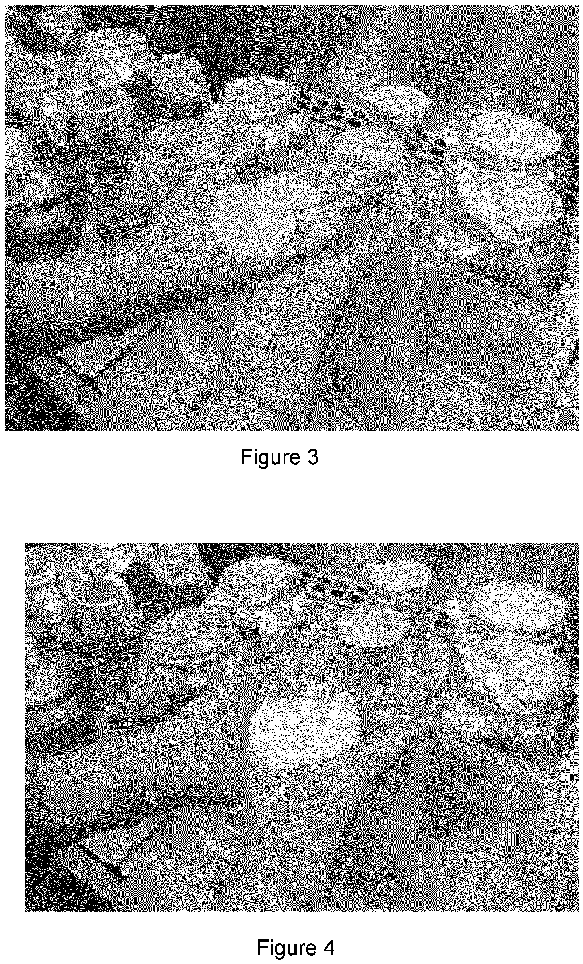

*Pleurotus ostratus* formed a monolayer of hyphae but the material strength was weak and easy to break (see Figure 3). The layer of entangled mycelium was clearly matching the shape of the flask base. When the biomass was dried, it yielded a final biomass concentration of 3 g (dry weight) / I of culture. The final material was free of substrate debris.

### Example 3 (using Ganoderma lucidum):

In this example, a detailed procedure for growing *Ganoderma lucidum* (oyster mushroom) using static submerged fermentation in Erlenmeyer flasks with a liquid medium is described.

### Materials and equipment:

- *Ganoderma lucidum* spores or mycelium culture.
- Erlenmeyer flasks (250 mL or 500 mL).
- Glucose.
- Citric acid monohydrate.
- ZnSO4·7H2O.
- Fe(NH4)2(SO4)2·6H2O.
- CuSO4·5H2O.
- MnSO4·4H2O.
- H3BO3.
- Na2MoO4·2H2O.
- Distilled water.
- Autoclave or pressure cooker.
- pH meter.
- Incubator.

### Procedure:

1. Preparation of liquid medium:
   - Weigh the following and add them to a beaker: 5 g of glucose, 5 g of citric acid monohydrate, 5 g of ZnSO4·7H2O, 1 g of Fe(NH4)2(SO4)2·6H2O, 0.25 g of CuSO4·5H2O, 0.05 g of MnSO4-4H2O, 0.05 g of H3BO3, 0.05 g of Na2MoO4·2H2O.
   - Add distilled water to make up to approximately 95 mL (up to 100 mL) to ensure all components are dissolved.
   - Adjust the pH of the medium to around 6.0 using a pH meter. Adjust with either HCl or NaOH as necessary.
   - Pour the prepared liquid medium into Erlenmeyer flasks, leaving some headspace to prevent overflow during sterilization.
2. Sterilization:
   - Cover the Erlenmeyer flasks with aluminum foil or cotton plugs.
   - Autoclave the flasks at 121°C for 15-20 minutes to sterilize the medium. Ensure that the medium is properly sterilized to prevent contamination.
3. Inoculation:
   - Allow the sterilized medium to cool down to room temperature in a sterile environment, such as a laminar flow hood.
   - Aseptically inoculate each flask with a small amount of *Ganoderma lucidum* spores or mycelium. Use a sterile loop or needle to transfer the inoculum to the medium.
4. Incubation:
   - Seal the flasks with sterile cotton plugs or aluminum foil.
   - Incubate the flasks in an incubator at the optimal temperature for Ganoderma lucidum growth, which is typically around 25-28°C.
5. Monitoring and growth assessment:
   - Periodically monitor the growth of *Ganoderma lucidum* in the liquid medium. Growth can be assessed visually by observing changes in the medium's appearance, such as mycelial growth, which may appear as white, cottony masses.

### Results:

*Ganoderma lucidum* formed a monolayer of hyphae, the material was resistant and rather dry compared to *Pleourotus ostratus* one (see Figure 4). The layer of entangled mycelium was clearly matching the shape of the flask base. When the biomass was dried, it yielded a final biomass concentration of 8 g (dry weight) / l of culture. The final material was free of substrate debris.

### Example 4 (using Pleourotus ostreatus):

In this case, a solid-state fermentation (SSF) procedure using Potato Dextrose Agar (PDA) media together with chickpea as the substrate is described. As already described above, SSF is a state of the art process.

### Materials and equipment:

- Pleourotus ostreatus spores or mycelium culture.
- Chickpeas (dried).
- Potato Dextrose Agar (PDA) plates.
- Autoclave or pressure cooker.
- Petri dishes.
- Glassware (flasks, beakers).
- Weighing scale.
- pH meter.
- Aluminum foil.
- Incubator.

### Procedure:

1. Preparation of chickpeas
   - Weigh the desired amount of chickpeas to be used as the solid substrate. Typically, it's around 100-150 grams per flask.
   - Rinse the chickpeas thoroughly under running water to remove any dust or contaminants.
   - Soak the chickpeas in water for 12-24 hours to hydrate them. Drain the excess water.
2. Sterilization of chickpeas
   - Transfer the soaked chickpeas to glass flasks, ensuring even distribution.
   - Seal the flasks with aluminum foil.
   - Autoclave or pressure cook the flasks at 121°C for 15-20 minutes to sterilize the chickpeas. This eliminates any competing microorganisms.
3. Inoculation:
   - Allow the sterilized chickpeas to cool down to room temperature, and pour them over a petri dish plate with sterile PDA media. Distribute the material evenly.
   - Using aseptic techniques, introduce *Pleourotus ostreatus* spores or mycelium culture to the chickpeas.
4. Incubation:
   - Seal the flasks with aluminum foil to maintain sterility.
   - Incubate the flasks in a temperature-controlled incubator at the optimal growth temperature for *Pleourotus ostreatus,* which is typically around 30 °C.
   - Ensure that the humidity level is appropriate for the fungus, around 70-80%. Moisture might need to be added periodically.
5. Monitoring:
   - Regularly check the progress of fungal growth. It may take several weeks for *Pleourotus ostreatus* to fully colonize the chickpea substrate.
   - Monitor the pH of the substrate to ensure it remains within the optimal range for the fungus, which is typically around pH 5.5-6.5. Adjust the pH if necessary.
6. Harvesting:
   - Once the chickpea substrate is fully colonized by *Pleourotus ostreatus,* it will appear white and myceliated.
   - Harvest the solid-state fermentation product by carefully removing the colonized chickpeas from the flasks. It will be used for further processing or extraction of desired compounds.

### Results:

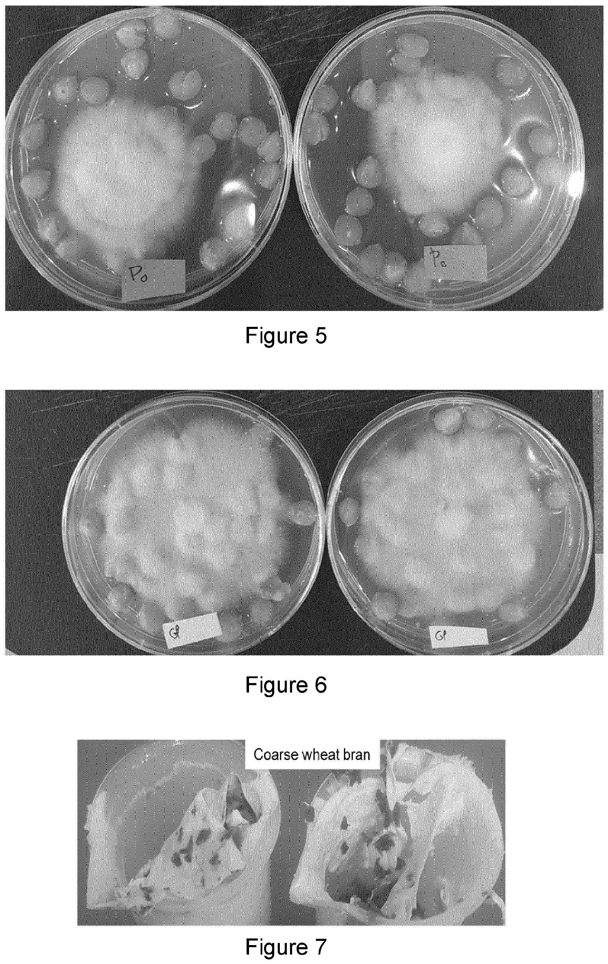

As seen in Figure 5, the fungus did not grow forming a filamentous fungus layer, which indicates that this procedure is not suitable for achieving a filamentous fungus biomaterial according to the invention.

### Example 5 (using Ganoderma lucidum):

In this case, a solid-state fermentation procedure using Potato Dextrose Agar (PDA) media together with chickpea as the substrate is described. As already described above, SSF is a state of the art process.

### Materials and equipment:

- *Ganoderma lucidum* spores or mycelium culture.
- Chickpeas (dried).
- Potato Dextrose Agar (PDA) plates.
- Autoclave or pressure cooker.
- Petri dishes.
- Glassware (flasks, beakers).
- Weighing scale.
- pH meter.
- Aluminum foil.
- Incubator.

### Procedure:

1. Preparation of chickpeas
   - Weigh the desired amount of chickpeas to be used as the solid substrate. Typically, it's around 100-150 grams per flask.
   - Rinse the chickpeas thoroughly under running water to remove any dust or contaminants.
   - Soak the chickpeas in water for 12-24 hours to hydrate them. Drain the excess water.
2. Sterilization of chickpeas
   - Transfer the soaked chickpeas to glass flasks, ensuring even distribution.
   - Seal the flasks with aluminum foil.
   - Autoclave or pressure cook the flasks at 121°C for 15-20 minutes to sterilize the chickpeas. This eliminates any competing microorganisms.
3. Inoculation:
   - Allow the sterilized chickpeas to cool down to room temperature, and pour them over a petri dish plate with sterile PDA media. Distribute the material evenly.
   - Using aseptic techniques, introduce *Ganoderma lucidum* spores or mycelium culture to the chickpeas.
4. Incubation:
   - Seal the flasks with aluminum foil to maintain sterility.
   - Incubate the flasks in a temperature-controlled incubator at the optimal growth temperature for *Ganoderma lucidum,* which is typically around 30 °C.
   - Ensure that the humidity level is appropriate for the fungus, around 70-80%. Moisture might need to be added periodically.
5. Monitoring:
   - Regularly check the progress of fungal growth. It may take several weeks for *Ganoderma lucidum* to fully colonize the chickpea substrate.
   - Monitor the pH of the substrate to ensure it remains within the optimal range for the fungus, which is typically around pH 5.5-6.5. Adjust the pH if necessary.
6. Harvesting:
   - Once the chickpea substrate is fully colonized by *Ganoderma lucidum,* it will appear white and myceliated.
   - Harvest the solid-state fermentation product by carefully removing the colonized chickpeas from the flasks. It will be used for further processing or extraction of desired compounds.

### Post-fermentation processing:

- Depending on the objectives, it is possible to further process the harvested, such as drying it for storage or extracting bioactive compounds.

### Results:

As seen in Figure 6, the fungus did not grow forming a filamentous fungus layer, which indicates that this procedure is not suitable for achieving a filamentous fungus biomaterial according to the invention.

### Example 6 (using Ganoderma lucidum):

In this case, a solid-state fermentation procedure using Potato Dextrose Agar (PDA) with wheat bran as the substrate is described. As already described above, SSF is a state of the art process.

### Materials and Equipment:

- *Ganoderma lucidum* spores or mycelium culture.
- wheat brans (dried).
- Potato Dextrose Agar (PDA) plates.
- Autoclave or pressure cooker.
- Petri dishes.
- Glassware (flasks, beakers).
- Weighing scale.
- pH meter.
- Aluminum foil.
- Incubator.

### Procedure:

1. Preparation of wheat brans
   - Weigh the desired amount of wheat brans to be used as the solid substrate. Typically, it's around 100-150 grams per flask.
   - Rinse the wheat brans thoroughly under running water to remove any dust or contaminants.
   - Soak the wheat brans in water for 12-24 hours to hydrate them. Drain the excess water.
2. Sterilization of wheat brans
   - Transfer the soaked wheat brans to glass flasks, ensuring even distribution.
   - Seal the flasks with aluminum foil.
   - Autoclave or pressure cook the flasks at 121°C for 15-20 minutes to sterilize the wheat brans. This eliminates any competing microorganisms.
3. Inoculation:
   - Allow the sterilized wheat brans to cool down to room temperature, and pour them into UV sterilized plastic beakers.
   - Using aseptic techniques, introduce *Ganoderma lucidum* spores or mycelium culture to the wheat brans.
4. Incubation:
   - Seal the flasks with aluminum foil to maintain sterility.
   - Incubate the flasks in a temperature-controlled incubator at the optimal growth temperature for *Ganoderma lucidum,* which is typically around 30 °C.
   - Ensure that the humidity level is appropriate for the fungus, around 70-80%. Moisture might need to be added periodically.
5. Monitoring:
   - Regularly check the progress of fungal growth. It may take several weeks for *Ganoderma lucidum* to fully colonize the wheat bran substrate.
   - Monitor the pH of the substrate to ensure it remains within the optimal range for the fungus, which is typically around pH 5.5-6.5. Adjust the pH if necessary.
6. Harvesting:
   - Once the wheat bran substrate is fully colonized by *Ganoderma lucidum,* it will appear white and myceliated.
   - Harvest the solid-state fermentation product by carefully removing the colonized wheat brans from the flasks. It will be used for further processing or extraction of desired compounds.

### Results:

As seen in Figure 7, even though the fungus grows in a layer form, wheat bran remains within the fungus layer, making it difficult to obtain a fungus layer free of debris. In the picture, the "little stains or spots" seen in the layer are wheat bran remains.

### Example 7 (chicken breast analogue recipe):

Ingredients:
- 82 grams of mycelium (grown according to the invention)
- 8.3 grams pea protein isolate
- 5 grams savoury seasoning blend (salt, pepper, garlic powder, among others.)

### Instructions:

1. Combine the mycelium obtained by the method according to the invention with pea protein isolate and seasoning blend.
2. Mix the ingredients until a homogenous mixture is achieved.
3. Shape the mixture into chicken breast-like portions.
4. Cook the analogues using preferred cooking methods (grilling, pan-searing, baking, etc.).
5. Serve the cooked chicken breast analogues as desired.

## Claims

1. Filamentous fungus biomaterial, **characterized in that** it comprises:
- less than 5% of culture media debris
- fungus filaments, an average length of said filaments being between 1 and 1,5 cm
- a water content comprised between 60 and 95%
- a thickness between 0.5 and 10 mm just after harvesting it.

2. Filamentous fungus biomaterial according to claim 1, **characterized in that** it comprises less than 1% of culture media debris.

3. A method for culturing a filamentous fungus, said method comprising the following steps:
a) providing a culture media, said culture media comprising a carbon source and a nitrogen source, wherein the ratio in dry weight between said carbon source and said nitrogen source is comprised between 0.5 and 50,
b) inoculating said culture media with a mycelium-based strain to obtain an inoculated media
c) conducting static submerged fermentation of said inoculated media in a sealed tray, wherein before fermentation starts a ratio between air volume and inoculated media volume in said tray is comprised between 0.025 and 0.12,
d) harvesting a filamentous fungus biomaterial.

4. Method according to claim 3, **characterized in that** said step c) of conducting static submerged fermentation is carried out at a temperature between 0 °C and 55 °C, preferably between 20 °C and 40 °C, even more preferably between 25 °C and 35 °C.

5. Method according to any one of claims 3 to 4, **characterized in that** said step c) of conducting static submerged fermentation is carried out for a duration of time comprised between 10 and 120 hours, preferably between 40 and 90 hours, even more preferably between 70 and 80 hours.

6. Method according to any one of claims 3 to 5, **characterized in that** a contact surface between said culture media and said air is between 1 and 3 cm²/ml of culture media.

7. Method according to any one of claims 3 to 6, **characterized in that** a thickness of said culture media is between 1 and 15 mm, preferably between 2.5 and 7.5 mm.

8. Method according to any one of claims 3 to 7, **characterized in that** said culture media comprises ingredients that enhance fungi growth, said ingredients being selected from the group comprising (NH4)2SO4, KH2P04, MgS04·7H2O, NaCl, and CaCl2·2H20.

9. Method according to any one of claims 3 to 8, **characterized in that** said culture media comprises insoluble particles, wherein at least 80% of said insoluble particles have a particle size of between 20 and 500 µm, preferably between 60 and 150 µm, and preferably said insoluble particles are complex nitrogen source particles.

10. Method according to any one of claims 3 to 9, **characterized in that** said mycelium-based strain is selected from an edible strain of the group consisting of *Ascomycota, Basidiomycota* and *Mucoromycota* divisions, preferably said mycelium-based strain is selected from an edible strain of the group consisting of *Aspergillus* and *Rhizopus.*

11. Method according to any one of claims 3 to 9, **characterized in that** said mycelium-based strain is selected from the group consisting of *Aspergillus oryzae, Aspergillus glaucus, Cordyceps sinensis, Fusarium venenatum (Quom), Ganoderma lucidum (Reishi), Laetiporus sulphureus, Monascus purpureus, Neurospora intermedia, Neurospora crassa, Penicillium nalgiovense, Penicillium chrysogenum, Rhizopus oryzae, Rhizopus chinensis, Rhizopus nigricans, Rhizopus oligosporus, Ustilago maydis, Agaricus campestris, Agaricus sylvaticus, Agrocybe aegerita (cylindracea), Amanita caesarea (open volva), Amanita ponderosa, Boletus aereus, Boletus edulis, Boletus pinophilus (pinicola), Boletus reticulatus (aestivalis), Calocybe gambosa, Cantharellus cibarius, Cantharellus cinereus, Cantharellus lutescens, Cantharellus tubaeformis, Clitocybe geotropa, Craterellus cornucioioides, Fistulina hepatica, Hygrocybe pratensis, Hydnum albidum, Hydnum repandum, Hydnum rufescens, Hygrophorus agathosmus, Hygrophorus gliocyclus, Hygrophorus latitabundus (limacinus), Hygrophorus marzuolus, Hygrophorus penarius, Hygrophorus russula, Lactarius delicious, Lactarius quietcolor, Lactarius salmonicolor, Lactarius sanguifluus, Lactarius semisanguifluss, Lepista panaeolus (luscina), Lepista nuda, Lepista personata, Macrolepiota procera, Marasmius oreades, Pleurotus eryngii, Pleurotus ostreatus, Rhizopogon luteolus (obtextus), Rhizopogon roseolus, Russula cyanozantha, Russula virescens, Suillus luteus, Terfezia arenaria, Terfezia claveryi, Terfezia leptoderma, Tricholoma portentosum, Tricholoma terreum, Tuber aestivum, Tuber borchii, Tuver brumale, Tuber indicum, Tuber magnatum, Tuber melanosporum (nigrum), Ustilago maydis, Xerocomus badius (Boletus badius), Agaricus arvensis, Agaricus bisporus, Agaricus bitorquis, agaricus blazei, Agaricus brunnescens, Agrocybe aegerita (cylindracea) Auricularia auriculaa-judae, Auricularia polytricha, Coprinus comatus, Flammulina velutipes, Grifola frondose, Hericium erinaceus, Lentinula erodes, Hypsizygus marmoreus, Hypsizygus tessulatus, Pholiota nameko, Pleurotus cystidiosus, Pleurotus cornucopiae (citrinopileatus), Pleurotus djamor, Pleurotus fabellatus, Pleurotus nebrodensis, Pleurotus pulmonarius, Pleurotus sajor-caju, Pleurotus tuber-regium, Sparassis crispa, Stropharia rugosoannulata, Tremella fuciformis, Tremella mesenterica, Tricholoma caligatum (matsutake), Volvariella volvacea, Helvella sp,* and *Morchella sp,* preferably said strain is selected from the group consisting of *Aspergillus oryzae, Aspergillus glaucus, Cordyceps sinensis, Ganoderma lucidum (Reishi), Monascus purpureus, Neurospora intermedia, Neurospora crassa, Penicillium nalgiovense, Penicillium chrysogenum, Rhizopus oryzae, Rhizopus chinensis, Rhizopus nigricans, Ustilago maydis, Agaricus bisporus (Portobello), Aspergilus orizae, Fusarium venenatum (Quom), Laetiporus sulphureus, Pleurotus ostreatus (oyster mushroom),* and *Rhizopus oligosporus.*

12. Mycelium-based meat analogue, **characterized in that** said meat analogue comprises, with respect of the total weight of said meat analogue:
- between 20 and 90% of a filamentous fungus biomaterial according to any one of claims 1 and 2;
- between 5 and 15 % of plant-based protein
- between 5 and 15 % of at least one texturing agent.

13. Mycelium-based meat analogue according to claim 12, **characterized in that** it is chicken meat analogue.

14. Meat analogue elaboration method, **characterized in that** it comprises the following steps:
a) combining a filamentous fungus biomaterial according to any one of claims 1 and 2 with at least one plant-based protein source and with at least one ingredient selected from the group consisting of non-starch polysaccharide, starch, vegetable oil and vegetable fat, to create a mycelium-based matrix
b) allowing said mycelium-based matrix to set, forming a mycelium-based meat analogue.

15. Method according to claim 14, **characterized in that** after step b) of allowing said mycelium-based matrix to set, a further step of cooking said mycelium-based matrix is performed at a temperature of between 60 and 95 °C.
